Europäisches Patentamt

European Patent Office

Office européen des brevets

(11) Veröffentlichungsnummer: **0 361 083**
**A1**

# EUROPÄISCHE PATENTANMELDUNG

(21) Anmeldenummer: 89115556.6

(22) Anmeldetag: 23.08.89

(51) Int. Cl.$^5$ **C07B 41/04 , C07C 43/11 , C07C 41/03**

(30) Priorität: 01.09.88 DE 3829751

(43) Veröffentlichungstag der Anmeldung:
04.04.90 Patentblatt 90/14

(84) Benannte Vertragsstaaten:
ES

(71) Anmelder: **Henkel Kommanditgesellschaft auf Aktien**
**Henkelstrasse 67**
**D-4000 Düsseldorf 13(DE)**

(72) Erfinder: **Ploog, Uwe, Dr.**
**Haydnweg 6**
**D-5657 Haan(DE)**

(54) Verwendung von Metallacetylacetonaten als Katalysatoren für die Ethoxylierung bzw. Propoxylierung.

(57) Die Verwendung von Metallacetylacetonaten der allgemeinen Formel I
$M(ACAC)_n$   (I)
in der
M ein Metallkation aus der von $Al^{3+}$, $Cr^{3+}$, $Fe^{3+}$, $Mn^{2+}$, $Sn^{2+}$, $Ti^{4+}$, $Zn^{2+}$ und $Zr^{4+}$ gebildeten Gruppe,
ACAC ein Anion des Acetylacetonats und
n eine Zahl entsprechend der Ladungszahl des Metallkations
bedeuten, als Katalysatoren für die Ethoxylierung bzw. Propoxylierung von Verbindungen mit aktiven H-Atomen ergeben eine enge homologen Verteilung der Alkoxylierungsprodukte.

EP 0 361 083 A1

## Verwendung von Metallacetylacetonaten als Katalysatoren für die Ethoxylierung bzw. Propoxylierung.

Die Erfindung betrifft Katalysatoren für die Ethoxylierung bzw. Propoxylierung von Verbindungen mit aktiven H-Atomen.

Unter Verbindungen mit aktiven H-Atomen im Sinne der Erfindung sind z.B. Fettalkohole, Guerbetalkohole, Fettsäuren und Amine zu verstehen, die bei der Ethoxylierung bzw. Propoxylierung nicht-ionische Detergentien bilden. Ein typisches Beispiel hierfür ist die Umsetzung von Fettalkoholen mit üblicherweise 10 bis 18 Kohlenstoffatomen mit Ethylenoxid und oder Propylenoxid in Gegenwart von Katalysatoren, wobei die Fettalkohole mit mehreren Molekülen Ethylenoxid und oder Propylenoxid reagieren.

Als Katalysatoren für die vorgenannte Polyalkoxylierung sind hierfür u.a. die folgenden eingesetzt worden:

Calcium- und Strontiumhydroxide, -alkoxide und phenoxide (EP-A 00 92 256),

Calciumalkoxide (EP-A 00 91 146),

Bariumhydroxid (EP-B 0 115 083),

basische Magnesiumverbindungen, z.B. Alkoxide (EP-A 00 82 569),

Magnesium- und Calciumfettsäuresalze (EP-A 0 85 167),

Antimonpentachlorid (DE-A 26 32 953),

Aluminiumisopropylat Schwefelsäure (EP-A 22 81 21),

Zink, Aluminium und andere Metalle enthaltende Oxoverbindungen (EP-A 180 266),

Aluminiumalkohole Phosphorsäuren (US 4 721 817).

Gebräuchliche Polyalkoxylierungskatalysatoren sind weiterhin Kaliumhydroxid und Natriummethylat.

Die vorgenannten Katalysatoren weisen u.a. den Nachteil auf. daß sie schlecht in das Reaktionssystem einarbeitbar und oder schwierig herstellbar sind. Weiterhin ist bei der Verwendung von Alkalihydroxiden sowie von Alkalialkoholaten die erreichbare Bandbreite des Polyalkoxylierungsgrades. d.h. die Homologenverteilung, auf die nachfolgend näher eingegangen wird, groß bzw. breit. Saure Katalysatoren, z.B. Antimonpentachlorid, bewirken zwar eine engere Homologenverteilung und niedrigeren Fettalkoholgehalt der Reaktionsprodukte, zeigen jedoch hohe Korrosivität und sind teilweise toxisch. Bei der Verwendung von Gemischen aus Aluminiumalkoholaten und schwefelsäure läßt sich zwar bei linearen Fettalkoholen eine enge Homologenverteilung der Alkoxylierungsprodukte bei gutem Umsatz erreichen, nicht jedoch bei verzweigten Alkoholen, z.B. den sogenannten Guerbet-Alkoholen.

Für Fettalkoholpolyalkoxylate ist die sogenannte enge Bandbreite des Polyalkoxylierungsgrades von besonderer Bedeutung, vgl. JAOCS, Vol. 63, 691 - 695 (1986), und HAPPI, 1986 (5), 52 - 54. Die sogenannten "narrow-range"-Alkoxylate weisen demnach insbesondere die folgenden Vorteile auf:

- niedrige Fließpunkte
- höhere Rauchpunkte
- weniger Mole Alkoxid zum Erreichen der Wasserlöslichkeit
- weniger Hydrotope für das Einbringen in flüssige Universalwaschmittel
- ein geringerer, durch Anwesenheit freier (nicht umgesetzter) Fettalkohole bedingter Geruch
- Reduzierung des Plumings beim Sprühtrocknen von Waschmittelslurries, die Fettalkoholpolyalkoxylat-Tenside enthalten.

Die Bandbreite bzw. Homologenverteilung der Fettalkoholpolyalkoxylate hängt wesentlich von der Art des verwendeten Katalysators ab. Als Maß für die Homologenverteilung gilt der sogenannte Q-Wert gemäß der Beziehung

$$Q = n^{\cdot} \cdot p^2$$

in der n⁻ die durchschnittliche Adduktzahl (mittlerer Ethoxylierungsgrad) und p den Prozentsatz des Adduktes mit bestimmten EO-Grad, das überwiegend gebildet wird, bedeuten. Ein großer Q-Wert bedeutet somit eine enge Bandbreite der Homologenverteilung.

Oxethylate mit einem niedrigen Gehalt an freien Fettalkoholen weisen bei der Herstellung von Ethercarbonsäuren durch Umsetzung der Oxethylate mit Natrium-chloracetaten in Gegenwart von Alkalihydroxiden einen erhöhten Umsetzungsgrad auf. Weiterhin sind Oxethylate mit eingeschränkter Homologenverteilung höchst wirksame Verdickungsmittel für Tensidlösungen.

Es wurde nun gefunden, daß unter erfindungsgemäßer Verwendung von Metallacetylacetonaten der allgemeinen Formel I

M(ACAC)$_n$   (I)

in der

M ein Metallkation aus der von Al$^{3+}$, Cr$^{3+}$, Fe$^{3+}$, Mn$^{2+}$, Sn$^{2+}$, Ti$^{4+}$, Zn$^{2+}$ und Zr$^{4+}$ gebildeten Gruppe,

ACAC ein Anion des Acetylacetonats und

n eine Zahl entsprechend der Ladungszahl des Metallkations

bedeuten, als Katalysatoren für die Ethoxylierung bzw. Propoxylierung von Verbindungen mit aktiven H-Atomen es zu einer überraschend engen homologen Verteilung der Polyalkoxylierungsprodukte kommt.

Zu den erfindungsgemäß einzusetzenden Metallacetylacetonaten der allgemeinen Formel I, die bekannte Verbindungen und größtenteils im Handel erhältlich sind, gehören:

$Al(ACAC)_3$

$Cr(ACAC)_3$

$Fe(ACAC)_3$

$Mn(ACAC)_2$

$Sn(ACAC)_2$

$Ti(ACAC)_4$

$Zn(ACAC)_2$

$Zr(ACAC)_4$.

Anstelle der vorgenannten Acetylacetonaten können auch Gemische von Salzen der vorgenannten Metalle mit Acetylaceton, welche die jeweiligen Acetylacetonate "in situ" bilden, verwendet werden, z.B. Zinksulfat/Acetylaceton- oder Zinn(II)oxalat/Acetylaceton-Gemische.

Besonders vorteilhaft sind Metallacetylacetonate aus der obigen Aufstellung, in denen das Metallkation M aus der von $Al^{3+}$, $Fe^{3+}$, $Zn^{2+}$ und $Zr^{4+}$ gebildeten Gruppen ausgewählt ist; derartige Metallacetylacetonate sind physiologisch unbedenklich und können daher, falls erwünscht, in den Alkoxylierungsprodukten verbleiben.

Gemäß einer bevorzugten Ausführungsform des Verfahrens der Erfindung setzt man die Katalysatoren in einer Menge von 0,1 bis 2 Gew.-%, bezogen auf das Endprodukt der Ethoxylierung bzw. Propoxylierung, zu.

Die Erfindung wird im folgenden anhand von bevorzugten Ausführungsbeispielen näher erläutert. Alle Prozentangaben sind in Gewichtsprozent.


1. Herstellung der Katalysatoren.

Die Reaktionsmischungen werden zweckmäßigerweise durch Vermischung der in Tabelle 1 aufgeführten Mengen an Acetylacetonaten mit dem zu polyalkoxylierenden Material, insbesondere den Fettalkoholen gemäß Tabelle 1, hergestellt. Man kann jedoch auch die Katalysatoren z.B. in Form einer konzentrierten Lösung in den Fettalkoholen herstellen, von der aliquote Teile zum Einsatz gelangen.

Anstelle der fertigen Katalysatoren können auch deren Ausgangsstoffe eingesetzt werden, z.B. ein Gemisch aus Zinksulfat-Heptahydrat und Acetylaceton (6 mMol/30 mMol pro 100 g Endprodukt der Ethoxylierung) und ein Gemisch aus Zinn(II)oxalat und Acetylaceton (10 mMol/30 mMol pro 100 g Endprodukt der Ethoxylierung).


2. Bedingungen der Oxalkylierung.

Die zu oxalkylierenden Fettalkohole gemäß Tabelle 1 wurden mit den angegebenen Katalysatoren versetzt und in einen geeigneten Autoklaven überführt. Nach ca. 30 min Evakuierung bei 100°C und Spülung mit Stickstoff wurde die Temperatur auf ca. 180°C gesteigert und die gewünschte Menge Ethylenoxid bei maximal 5 bar aufgedrückt. Nach einer Nachreaktionszeit von 30 min wurde nicht umgesetztes Alkylenoxid bei 100°C/14 mbar abgezogen.

Von den gebildeten Alkoxylaten wurden die OH-Zahl und der Polyglykolgehalt (PG) bestimmt; weiterhin wurde ein Gaschromatogramm zur Ermittlung der Homologenverteilung bzw. des Q-Wertes anhand der gemessenen Flächenprozente erstellt.


Beispiele 1 bis 4.

In diesen Beispielen wurde nach der obigen Arbeitsvorschrift zur Ethoxylierung von Fettalkoholen mit 2 Mol Ethylenoxid verfahren. In Tabelle 1 sind die eingesetzten Fettalkohole und Katalysatoren, die eingesetzte Katalysatormenge (in mMol/100 g Ethoxylierungsprodukt), die Reaktionszeit, die Reaktionstemperatur, der ermittelte Q-Wert, die OH-Zahlen der Ethoxylierungsprodukten (gefunden/berechnet), der Gehalt an freien Fettalkoholen (FFA, %) und der Polyglykolgehalt (PG, %) zusammengefaßt.

3

Vergleichsversuche.

Die Ergebnisse mit aus dem Stand der Technik bekannten Katalysatoren sind in Tabelle 2 zusammengefaßt.

Die in den Tabellen erwähnten Fettalkohole waren handelsübliche, technische Fettalkoholgemische der folgenden Zusammensetzung:

C 12.14: Fettalkohol der Kettenlängenverteilung 0 - 2 % C 10, 70 - 75 % C12, 20 - 30 % C 14, 0 - 2 % C 16; Jodzahl kleiner als 0.3, durchschnittliches Molekulargewicht 194, Hydroxylzahl 285 - 293.

C 12: Fettalkohol der Kettenlängenverteilung 0 - 2 % C 10, mindestens 97 % C 12, 0 - 3 % C 14, Jodzahl kleiner als 0.3, durchschnittliches Molekulargewicht 188, Hydroxylzahl 295 -301.

Tabelle 1

| Ethoxylierung von Alkoholen mit 2 Mol Ethylenoxid | | | | | | | | |
|---|---|---|---|---|---|---|---|---|
| Beispiel Nr. | Alkohol | Katalysator | Kat.menge.mMol. 100 g Produkt | Rkt.zeit (h).-temp. ($^{\circ}$C) | Q | OH-Zahl gef. ber. | FFA (%) | PG (%) |
| 1 | C 12 | Al (ACAC)$_3$ | 3,1 | 180.6 | 2312 | 205.204 | 18 | 1 |
| 2 | C 12 | Fe(ACAC)$_3$ | 3,0 | 150.6 | 1624 | 206.204 | 15 | 0.9 |
| 3 | C 12 | Zr(ACAC)$_4$ | 3,0 | 150.5 | 1800 | 207.204 | 19,5 | 1,4 |
| 4 | C 12 | Zn(ACAC)$_2$ | 3,5 | 150-180.5 | 3000 | 211.204 | 17,1 | 0,7 |

Tabelle 2

| Ethoxylierung von Alkoholen mit 2 Mol Ethylenoxid (Vergleichsversuche) | | | | | | | |
|---|---|---|---|---|---|---|---|
| Fettalkohol | Katalysator | Kat.menge (mMol 100 g Produkt) | Rkt.zeit (h).-temp. ($^{\circ}$C) | Q | OH-Zahl gef. ber. | FFA (%) | PG (%) |
| C 12.14 | NaOCH$_3$ | 2 | 180.1 | 655 | 204.204 | 31,9 | 0,8 |
| C 12 | SbCl$_5$ | 1,7 | 180.5 | 1972 | 208.204 | 8,3 | 1.2 |

**Ansprüche**

1. Verwendung von Metallacetylacetonaten der allgemeinen Formel I

M(ACAC)$_n$     (I)

in der

M ein Metallkation aus der von Al$^{3+}$, Cr$^{3+}$, Fe$^{3+}$, Mn$^{2+}$, Ni$^{2+}$, Sn$^{2+}$, Ti$^{4+}$, Zn$^{2+}$ und Zr$^{4+}$ gebildeten Gruppe,

ACAC ein Anion des Acetylacetonats und

n eine Zahl entsprechend der Ladungszahl des Metallkations

bedeuten, als Katalysatoren für die Ethoxylierung bzw. Propoxylierung von Verbindungen mit aktiven H-Atomen.

2. Verwendung nach Anspruch 1, dadurch gekennzeichnet, daß man Metallacetylacetonate der allgemeinen Formel I einsetzt, in der M ein Metallkation aus der von Al$^{3+}$, Fe$^{3+}$, Zn$^{2+}$ und Zr$^{4+}$ gebildeten Gruppe ist und ACAC sowie n wie oben definiert sind.

3. Verwendung nach Anspruch 1 oder 2, dadurch gekennzeichnet, daß man die Katalysatoren in einer Menge von 0,1 bis 2 Gew.-%, bezogen auf das Endprodukt der Ethoxylierung bzw. Propoxylierung, einsetzt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 034 648 (CONOCO)<br>* Ansprüche; Beispiel 18 *<br>--- | 1-3 | C 07 B 41/04 ✓<br>C 07 C 43/11<br>C 07 C 41/03 |
| D,A | EP-A-0 180 266 (SHELL INTERNATIONALE RESEARCH)<br>* Ansprüche; Beispiele *<br>--- | 1-3 | |
| A | FR-A-2 218 343 (PRODUITS CHIMIQUES UGINE KUHLMANN)<br>* Beispiel 22 *<br>----- | 1-3 | |
| | | | **RECHERCHIERTE SACHGEBIETE (Int. Cl.5)**<br><br>C 07 B 41/00<br>C 07 C 41/00<br>C 07 C 43/00 |

Der vorliegende Recherchenbericht wurde für alle Patentansprüche erstellt

| Recherchenort | Abschlußdatum der Recherche | Prüfer |
|---|---|---|
| DEN HAAG | 10-11-1989 | WRIGHT M.W. |

EPO FORM 1503 03.82 (P0403)